(19) Europäisches Patentamt
European Patent Office
Office européen des brevets

(11) Publication number: **0 005 288**
**A1**

(12) **EUROPEAN PATENT APPLICATION**

(21) Application number: **79200181.0**

(22) Date of filing: **12.04.79**

(51) Int. Cl.[2]: **C 07 C 97/07**, A 01 N 9/20, C 07 C 103/42, C 07 C 147/06, C 07 C 147/12, C 07 D 295/10

(30) Priority: **21.04.78 US 898575**

(43) Date of publication of application: **14.11.79 Bulletin 79/23**

(84) Designated Contracting States: **BE DE FR GB IT NL**

(71) Applicant: **CHIMAC SOCIETE ANONYME, 52 Rue de l'Etuve, B-1000 Bruxelles (BE)**

(72) Inventor: **Goldblatt, André J., 7 Avenue du Domaine, B-1190 Bruxelles (BE)**
Inventor: **Forni, Luciano, 49 Résidence du Dr. Cambier, B-7111 Saint-Vaast/La Louviere (BE)**
Inventor: **Gillet, André J., 5 Avenue de l'Arc d'Airain, B-5800 Gembloux (BE)**

(74) Representative: **DE BRABANTER, Maurice et al, Bureau VANDER HAEGHEN 63 Avenue de la Toison d'Or, B-1060 Bruxelles (BE)**

(54) **Derivatives of 1-amino-fluoren-9-one and their use as herbicides.**

(57) This invention relates to new derivatives of 1-amino-fluoren-9-one and their use as herbicides.

The new compounds may be represented by the formula:

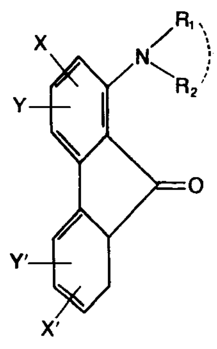

in which $R_1$ and $R_2$, which may be identical or different, represent a lower alkyl, alkanoyl, carbalcoxy, cycloalkanecarbonyl, alkenyl or alkynyl radical, which may be substituted by a halogen atom and may also form with the attached nitrogen atom a heterocyclic ring, whereas one or two of the symbols $R_1$ and $R_2$ represent hydrogen, X and X', which may be identical or different, represent hydrogen, a halogen atom, a trihalogenomethyl radical or a trifluoromethylsulfonyl ($CF_3SO_2$) radical, with the proviso that X and X' do not each represent hydrogen, and Y and Y', which may be identical or different, represent hydrogen or a nitro radical.

**EP 0 005 288 A1**

Derivatives of 1-amino-fluoren-9-one and their use as herbicides

This invention relates to new derivatives of 1-amino-fluoren-9-one and to the preparation thereof.

It also relates to new preemergence herbicidal compositions containing at least one new derivative of 1-amino-fluoren-9-one.

The new derivatives of 1-amino-fluoren-9-one of the present invention may be represented by the following formula :

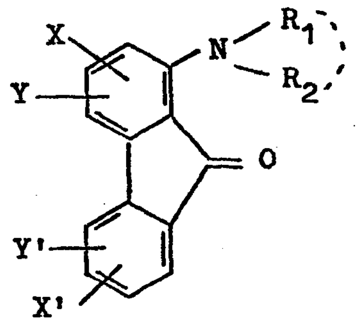

(I)

in which $R_1$ and $R_2$ which may be identical or different, represent a lower alkyl, alkanoyl, carbalcoxy, cycloalkanecarbonyl, alkenyl or alkynyl radical which may be substituted by a halogen atom and may also form with the attached nitrogen atom a heterocyclic ring, whereas one or two of the symbols $R_1$ and $R_2$ may also represent hydrogen, X and X' which may be identical or different represent hydrogen, a halogen atom such as chlorine, bromine or fluorine, a trihalogenomethyl radical, such as trifluoromethyl, trichloromethyl or tribromomethyl radical, or a trifluoromethylsulfonyl ($CF_2SO_2$) radical, with the proviso that X and X' do not each represent hydrogen and Y and Y' which may be identical or different, represent hydrogen or a nitro radical.

This invention relates particularly to new derivatives of 1-amino-fluoren-9-one in which $R_1$ and $R_2$, which may be identical or different, represent hydrogen or a lower alkyl radical and X and X' which may be identical or different, represent hydrogen or a halogen atom, with the proviso that X and X' do not each represent hydrogen and Y as well as Y' represent hydrogen.

Among the new derivatives of 1-amino-fluoren-9-one of formula I, the following may be cited by way of examples, other compounds according to this invention being described in examples 5 to 37 :

- 1-dimethylamino-2-fluoro-fluoren-9-one ;
- 1-amino-2-fluoro-fluoren-9-one ;
- 1-dimethylamino-4-fluoro-fluoren-9-one ;
- 1-methylamino-4-fluoro-fluoren-9-one ;
- 1-amino-4-fluoro-fluoren-9-one ;
- 1-dimethylamino-7-fluoro-fluoren-9-one.

The compounds according to this invention may be prepared as follows.

When, in the formula I, $R_1$ and $R_2$ represent an alkyl, alkanoyl, carbalcoxy, cycloalkanecarbonyl, alkenyl or alkynyl radical, the compounds of formula I may be prepared by reacting a 1-amino-fluoren-9-one of the following formula :

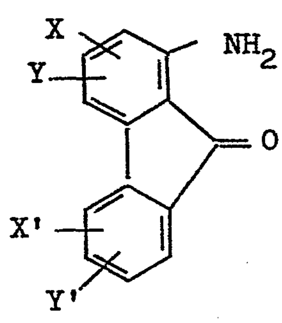

(II)

in which X, X', Y and Y' have the above meanings, with an alkylating, alkanoylating, carbalcoxylating, cycloalkane-carbonylating, alkenylating or alkynylating reagent.

For example, when it is desired to obtain a compound of formula I, in which $R_1$ and $R_2$ represent the same alkyl radical, whereas X, X', Y and Y' have the above meanings,

the compound of formula II may be reacted with an alkyl p-toluene sulfonate or a dialkyl sulfate.

When, in the compounds of formula I, $R_1$ represents hydrogen and $R_2$ represents a lower alkyl, alkanoyl, carbalcoxy, cycloalkanecarbonyl, alkenyl or alkynyl radical, the compounds of formula I may also be prepared by reacting a compound of the following formula :

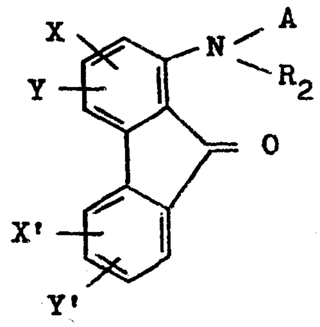

(III)

in which $R_2$ represents a lower alkyl, alkanoyl, carbalcoxy, cycloalkanecarbonyl, alkenyl or alkynyl radical, X, X', Y and Y' have one of the above meanings and A represents an alkanoyl group such as an acetyl group, an alkoxycarbonyl or carbalcoxy or cycloalkanecarbonyl group or a toluenesulfonyl (tosyl) group, with sulfuric acid.

It has been found that the compounds of formula I have remarkable preemergence and postemergence herbicidal properties.

This invention therefore relates also to the preemergence and postemergence herbicidal compositions containing an effective amount of at least one compound of formula I.

The herbicidal compositions according to this invention may be solid or liquid formulations consisting of at least one compound of formula I and an inert carrier or conventional formulation adjuvant.

Preparation of said compositions broadly involves admixing an effective amount of the herbicidal agent of formula I and adjuvant.

Use of said compositions broadly involves application of an effective amount of said compounds of formula I or preferably said herbicidal compositions to the soil

containing seeds of the plants or plants to be controlled.

Typical formulations include, for instance, dusts, wettable powders, granules, emulsionable concentrates, free flowable concentrates and the like.

Dusts are generally prepared by grinding together from about 2% to 98% by weight of at least one active material of formula I with from about 98% to 2% by weight of a solid diluent, such as attaclay, kaolin, diatomaceous earth, fullers earth, silica, talc, pumice, or the like.

Granular formulations may be prepared by applying a liquid solution or suspension of the active material to sorptive granular carriers, such as attaclay, kaolin or diatomite granules. Alternatively, they may be mixed with inert carriers and applied to non-sorptive granules, such as sand or limestone.

Wettable powders are prepared by grinding at least one active ingredient of formula I with a solid carrier, such as used in the dust formulation.

In addition to the solid carriers, the wettable powders also contain a dispersing agent and/or a surfactant or wetting agent, such as sodium lignosulfonate and/or a condensate of polyoxyethylene with an alcohol or an alkylphenol, namely nonylphenol.

The wettable powders are usually dispersed in water and applied as a liquid spray to the area or locus where control of undesirable plant species is desired.

The emulsionable concentrate is prepared by dissolving a compound of formula I in a liquid carrier such as a mixture of xylenes containing a surfactant and a wetting agent such as calcium dodecylbenzenesulfonate and a condensate of polyoxyethylene with an alcohol or an alkylphenol, namely nonylphenol.

The emulsionable concentrate is usually dispersed in water and applied as a liquid spray to the area or locus where control of undesirable plant species is necessary.

For use as preemergence or postemergence herbicides, the dusts or liquid sprays containing the active compound

of formula I can be applied to the soil shortly prior to or after planting or very shortly after the emergence of weeds. In some cases, the active herbicidal compounds of formula I could be advantageously used in mixture with known herbicides.

This invention also relates to a method for the pre- and postemergence control of undesirable plant species, said method consisting in applying to soil containing seeds of said undesirable plant species a herbicidally effective amount of at least one compound of formula I. The effective amount of said compounds is of about 0.5 to 6 kg/hectare, preferably about 2.5 kg/hectare.

EXAMPLES

The preparation of the new compounds of formula I is represented by the following reaction scheme and illustrated by the following non-limitative examples 1 to 37.

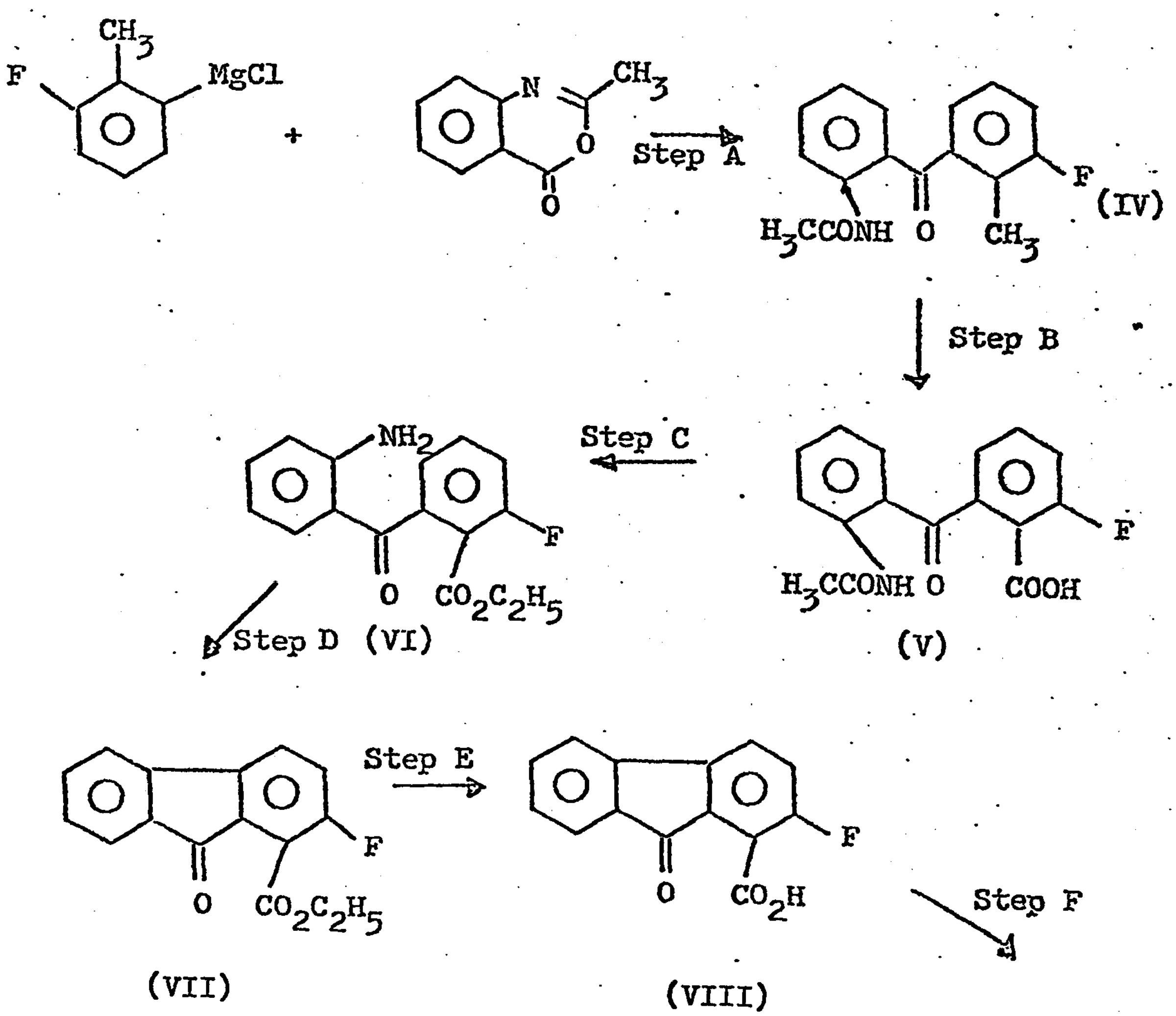


(IV) (V) (VI) (VII) (VIII)

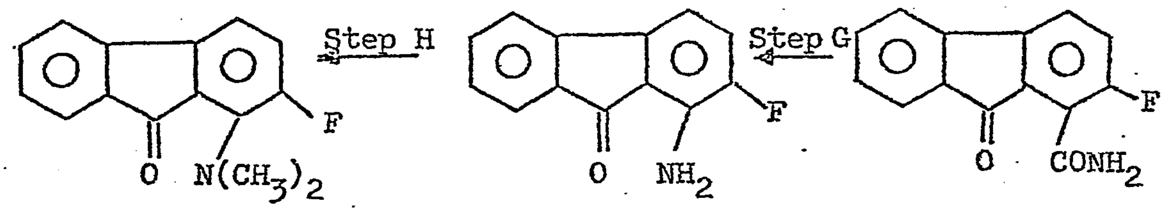


(XI) (X) (IX)

The identification characteristics of the various compounds disclosed in the examples have been obtained under the following conditions.

All analysis stand within the normal standards.

Melting points are uncorrected and are measured on a Boetius hot-stage microscope.

IR spectra are measured on a Pye-Unicam SP 1100 spectrophotometer and are recorded from KBr discs.

Mass spectra (MS) are recorded on a Jeol JMS-D 100 MARK II double-focusing mass-spectrometer at 75 eV.

NMR spectra are recorded on a Varian T 60 spectrometer with TMS as internal standard. The solvent is indicated with brackets.

Example 1:1-dimethylamino-2-fluoro-fluoren-9-one(P 233)

Step A : 2'-methyl-3'-fluoro-2-acetylamino-benzophenone (IV)

10 ml of dry tetrahydrofuran, 0.5 g of Mg and one crystal of iodine are refluxed until complete discoloration of the medium. 0.5 ml of ethyl bromide is added to initiate the reaction.

Then, 2.9 g of 2-chloro-6-fluoro-toluene are added dropwise.

Reflux is maintained until almost all the magnesium is consumed.

After cooling at room temperature, the mixture is filtered in a dropping funnel. The resulting filtrate is added dropwise to a stirred suspension of 3.2 g of acetylanthranil (prepared by the method of L.A. Errede and J.J. McBrady, J. Org. Chem., 42, 3863 (1977)) in 20 ml of dry tetrahydrofuran at -10°C, the temperature being held below

-5°C. After the addition is completed, the stirring is continued for 1/2 hour at -5°C, 1/4 hour at 0°C and one hour at room temperature.

The mixture is cooled to -10°C and 25 ml of HCl 2N are added dropwise, the temperature being maintained below 0°C. The resulting suspension is extracted three times with 50 ml of ether. The combined ethereal extracts are washed once with 20 ml of NaOH 0.5N, twice with water, dried on $MgSO_4$ and evaporated to dryness. The resulting oil is extracted with hexane. Upon evaporation of the solvent, the product crystallises : Yield 33% (m.p. 113-114°C).

I.R. : $\nu_{CO}$ = 1695 $cm^{-1}$

NMR : ($CDCl_3$) : $C\underline{H}_3$ : doublet - 2.20 $\delta$ - 3H
$COC\underline{H}_3$ : singlet : 2.30 $\delta$ - 3H
$N\underline{H}$ : broad - 11.5 $\delta$ - 1H

MS : $M^{+\cdot}$ = m/e 271.

Step B : 2'-acetylamino-3-fluoro-benzophenone-2-carboxylic acid

1 g of 2-methyl-3-fluoro-2'-acetylamino-benzophenone is added to a refluxing solution of 1 g of $MgSO_4$ in 50 ml of water. 2 g of $KMnO_4$ are added portionwise. The reflux is maintained for 5 hours. While hot, the mixture is filtered and the filtrate acidified with HCl 2N and left cooling to room temperature. The resulting crystals are filtered and dried. Yield : 39% (m.p. 178.5-180°C).

I.R. : $\nu_{CO}$ = 1715 $cm^{-1}$

NMR : (DMSO-$d_6$) : $COC\underline{H}_3$ : singlet - 2.05 $\delta$ - 3H
$COO\underline{H}$ : broad - 10.50 $\delta$ - 1H (exchanges with $D_2O$)

MS : $M^{+\cdot}$ = m/e 301.

Step C : Ethyl 2'-amino-3-fluoro-benzophenone-2-carboxylate (VI)

16 g of 2'-acetylamino-3-fluoro-benzophenone-1-carboxylic acid are dissolved in a saturated solution of HCl gas in absolute ethanol. The mixture is heated 20 h at 40°C.

After cooling, the excess alcohol is evaporated under vacuum. The residual oil is dissolved in chloroform,

washed three times with 50 ml of NaOH 1N and once with water.

The dried organic layer is evaporated to dryness. The ester is obtained as an oil that crystallises slowly. The crystals are recrystallized from cyclohexane. Yield 98% (m.p. : 85-86°C).

IR : $\nu_{CO}$ = 1720 & 1725 $cm^{-1}$

$\nu_{NH}$ = 3360 - 3480 $cm^{-1}$

NMR : ($CDCl_3$) : $CH_2\underline{CH_3}$ : triplet - 1.10$\delta$ - 3H

$\underline{CH_2}CH_3$ : quadruplet - 4.12$\delta$ - 2H

$\underline{NH_2}$ : singlet - 4.65$\delta$ -1H (exchanges with $D_2O$)

MS : $M^{+\cdot}$ = m/e 287.

<u>Steps D, E, F and G :</u>

<u>1-amino-2-fluoro-flucen-9-one</u> (X)

All the following steps may be performed without purification of the intermediate products. Global yield of the steps D to G is 15.4%.

<u>Step D</u> : <u>Ethyl 2-fluoro-fluoren-9-one-1-carboxylate</u>(VII)

48 g of ethyl 2'-amino-3-fluoro-benzophenone-2-carboxylate are dissolved in 840 ml of $H_2SO_4$ 50% and cooled to -5°C. A saturated solution of 13.7 g of $NaNO_2$ in water is added dropwise, maintaining the temperature at -5°C. After the addition is completed, the solution is stirred 1/2 hour, at 0°C, one hour at room temperature and finally two days at 50°C. A yellow precipitate forms slowly. The mixture is poured on 1 l. of water. The resulting solid is filtered and washed with water until neutrality of the filtrate is achieved. The characteristics of the pure product isolated from column chromatography on silica gel (eluent : $CHCl_3$) are the following :

m.p. 122-123°C

IR : $\nu_{CO}$ : 1715 & 1730 $cm^{-1}$

NMR : ($CDCl_3$) : $CH_2\underline{CH_3}$ : triplet 1.45$\delta$ - 3H

$\underline{CH_2}CH_3$ : quadruplet - 4.55$\delta$ - 2H

MS : $M.^{+}$ = m/e 270.

Step E : 2-fluoro-fluoren-9-one-1-carboxylic acid (VIII)

The ethyl 2-fluoro-fluoren-9-one-1-carboxylate is mixed with 500 ml of NaOH 6N and refluxed for 2 hours. After cooling, the mixture is diluted with twice its volume of water and acidified with concentrated HCl. The yellow precipitate that forms is filtered and washed with water. The melting point of the pure product isolated from column chromatography on silica gel (eluent : $CHCl_3/CH_3CO_2C_2H_5$ 3/1) is 209-210°C.

Step F : 2-fluoro-fluoren-9-one-1-carboxamide (IX)

The well dried 2-fluoro-fluoren-9-one-1-carboxylic acid is refluxed for 2 hours with 500 ml of $SOCl_2$. After reaction, the excess of $SOCl_2$ is evaporated under vacuum, the last traces of the reactant being eliminated by azeotropic distillation twice with benzene. The resulting solid is dissolved in 300 ml of dry benzene and 200 ml of concentrated $NH_4OH$ are added. Upon stirring at room temperature, a yellow precipitate appears. The stirring is maintained for 2 hours. The solid is filtered and washed with water until the filtrate is neutral. The product, recrystallized from acetic acid, has the following melting point : 220°C (dec.).

Step G : 1-amino-2-fluoro-fluoren-9-one (IX) (P 221)

The 2-fluoro-fluoren-9-one carboxamide is added portionwise to 165 ml of a solution of NaOBr at 0°C (obtained by the reaction of 5 ml of $Br_2$ and 160 ml of NaOH 3N).

The mixture is stirred 15 minutes at 0°C and then at 10°C until a clear solution is obtained. The solution is then heated stepwise to 80°C. A yellow precipitate appears. The heating is maintained until the evolution of gas ceases. The mixture is poured on 200 ml of water. The solid is filtered, washed with water and dried.

(m.p. 130-131°C).

I.R. : $\nu_{CO}$ : 1695cm$^{-1}$ $\nu_{NH2}$ : 3350-3450 cm$^{-1}$

NMR : ($CDCl_3$) : $NH_2$ : broad - 5.55$\delta$ - 2H (exchanges with $D_2O$)

MS : $M^{+\cdot}$ = m/e 213.

Step H : 1-dimethylamino -2-fluoro-fluoren-9-one (XI) (P 233)

8 g of 1-amino-2-fluoro-fluoren-9-one are refluxed with stirring with 160 ml of freshly distilled $(CH_3)_2SO_4$. After completion of the reaction (2 hours), the excess of sulphate is evaporated under vacuum until only an oily residue remains. The oil is poured on 200 ml of water and basified with concentrated $NH_4OH$ with vigourous stirring. The solution is extracted exhaustively with $CHCl_3$. The combined organic layers are dried and evaporated to dryness.

8.5 g (93.9%) of an oil crystallises.

M.P. 45-46°C.

I.R. : $\nu_{CO}$ = 1695 $cm^{-1}$

NMR : ($CDCl_3$) : $CH_3$ : double singlet - 3.10 and 3.15 $\delta$ - 2 x 3H

M.S. : $M^{+\cdot}$ = m/e 241.

Examples 2 to 4

The following compounds have been prepared by the method described in example 1 :

- 1-dimethylamino-4-fluoro-fluoren-9-one (M.P. 105-106°C)(P180)
- 1-methylamino-4-fluoro-fluoren-9-one (M.P. 139-144°C)(P 182)
- 1-amino-4-fluoro-fluoren-9-one (M.P. 205-206°C) (P 318).

Example 5

1-di-N-propylamino-2-fluoro-fluoren-9-one (P 325)

A mixture of 20 g of 1-amino-2-fluoro-fluoren-9-one and of 60 g of n-propyl toluene-p-sulfonate

(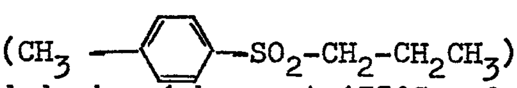)

is heated during 1 hour at 135°C and thereafter during one hour at 165°C.

After cooling to room temperature, 140 ml of methanol are added and the mixture is refluxed. When the reflux temperature has been obtained, 140 ml of a 10% solution of $CH_3ONa$ in methanol are added.

While hot the reaction mixture is poured into 500 ml of water. The aqueous phase separated from an oily brown phase is extracted with methylene dichloride and the methylene dichloride extract is washed with water, dried and evaporated to dryness.

The residue is chromatographied on silica (eluent : benzene).

9.32 g of 1-di-n-propylamino-4-fluoro-fluoren-9-one are obtained. Yield : 30.5% ; M.P. 69-71°C.

Examples 6 to 12

The following compounds have been prepared by the process described in example 5 :

- 1-dimethylamino-2-fluoro-fluoren-9-one (M.P. 45-46°C) (P 233)
- 1-di-n-butylamino-2-fluoro-fluoren-9-one (oil) (P 339)
- 1-diethylamino-2-fluoro-fluoren-9-one (oil) (P 353)
- 1-dimethylamino-4-fluoro-fluoren-9-one (M.P. 135.5-136.5°C) (P 350)
- 1-dimethylamino-4-fluoro-9-fluoren-9-one (M.P. 105-106°C) (P 180)
- 1-amino-7-fluoro-fluoren-9-one (M.P. 150-152°C)
- 1-dimethylamino-7-fluoro-9-fluoren-9-one (M.P. 108.5-110°C) (P 209)

Example 13

1-ethylamino-2-fluoro-fluoren-9-one (P 351)

To a solution of 12 g of 1-amino-2-fluoren-9-one in 120 ml of ethyl orthoformate, 0.6 g of concentrated sulfuric acid are added.

The reaction mixture is heated at 120°C and the formed ethanol is removed by distillation. When the stoechiometric amount of ethanol has been distilled, the temperature of the reaction mixture is increased to 175°C to remove the major part of the excess of orthoformate.

The inner temperature of the reactor in which the reaction takes place is then maintained at 145°C during 90 minutes and thereafter the mixture is distilled under vacuum to dryness.

The brown residue is treated with 240 ml of 6N HCl at the reflux temperature and the medium is maintained at this temperature until the oil has completely disappeared. While hot the reaction mixture is poured into 1 litre of water.

The aqueous phase is extracted with ethyl acetate and the organic phase is washed with water, dried and evaporated to dryness. The obtained oil becomes solid and

is crystallized from a mixture of ethanol and water. 9 g of yellow needles are obtained. Yield : M.P. 93.5-95°C.

Examples 14 to 21

The following compounds have been prepared by the method described in example 13 :

- 1-methylamino-4-fluoro-fluoren-9-one (M.P. 139-144°C)(P 327)
- 1-n-propylamino-2-fluoro-fluoren-9-one (M.P. 56-57°C)(P 325)
- 1-(N-methyl-N-formyl)amino-2-fluoro-fluoren-9-one(M.P. 158-159°C) (P 326)
- 1-methylamino-2-fluoro-fluoren-9-one (M.P. 137-138°C) (P 327)
- 1-n-butylamino-2-fluoro-fluoren-9-one (oil) (P 348)
- 1-ethylamino-2-fluoro-fluoren-9-one (M.P. 94-95°C) (P 351)
- 1-isopropylamino-2-fluoro-fluoren-9-one (M.P. 65.5-66.5°C) (P 362)
- 1-chloroethylamino-2-fluoro-fluoren-9-one (M.P. 122-123°C) (P 365)

Example 22

1-trifluoroacetylamino-2-fluoro-fluoren-9-one (P 331)

To a suspension of 20 g of 1-amino-2-fluoro-fluoren-9-one in 200 ml of anhydrous carbon tetrachloride, 29.6 g of trifluoracetic anhydride are added drop by drop at room temperature.

The reaction mixture is stirred during 30 minutes at room temperature and then poured on ice.

The aqueous phase is extracted with ethyl acetate and the organic phase is washed with water, dried and evaporated to dryness. 27 g of a yellow solid are obtained. After crystallization from a mixture of methanol and water, the final product melting at 187-188°C is obtained with a yield of 93%.

Example 23

Using the process described in example 22, the following compound is prepared :

- 1-diacetylamino-2-fluoro-fluoren-9-one (M.P. 151-152°C) (P 352).

Example 24

1-acetylamino-2-fluoro-fluoren-9-one (P 323)

To a mixture of 20 ml of anhydrous benzene and 0.5 g of iodine, a solution of 0.36 g of acetyl chloride in

anhydrous benzene (5 ml) is added. The reaction mixture is heated to reflux during 6 hours and then poured on ice. The aqueous phase is extracted with chloroform and the organic phase is washed with water, dried and evaporated to dryness. The obtained yellow product is recrystallized from a mixture of methanol and water. Yellow needles (0.34 g) melting at 196-197°C are obtained with a yield of 54%.

Examples 25 to 29

Using the process described in example 25, the following compounds are prepared :

- 1-acetylamino-4-fluoro-fluoren-9-one (M.P. 180.5-181°C) (P 207)
- 1-chloroacetylamino-2-fluoro-fluoren-9-one (M.P. 184-185°C) (P 334)
- 1-cyclopropylcarbonylamino-2-fluoro-9-fluoren-9-one (M.P. 203-204°C) (P 344)
- 1-chloroacetylamino-4-fluoro-fluoren-9-one (M.P. 177.5-178°C) (P 194)
- 1-éthoxycarbonylamino-4-fluoro-9-fluoren-9-one (M.P. 156.5-158°C) (P 223).

Example 30

1-(N-methyl-N-trifluoroacetylamino)-2-fluoro-fluoren-9-one (P 333)

10 g of trifluoroacetylamino-2-fluoro-fluoren-9-one dissolved in 70 ml of dimethylformamide are added drop by drop to a suspension of 0.77 g of sodium hydride in 30 ml of dimethylformamide.

To the obtained solution, 4.69 g of methyl iodide are added drop by drop and the reaction mixture is maintained during 30 minutes at room temperature and then cautiously poured on ice.

The aqueous phase is extracted with ethyl acetate and the organic phase is washed with water, dried and evaporated to dryness. The obtained oil is crystallized from a mixture of methanol and water. The desired product (5.21 g) melts at 123-124°C. Yield : 50%.

Examples 31 to 34

The following compounds are prepared by the method described in example 30 :

- 1-(N-n-propyl-N-cyclopropyl)amino-2-fluoro-fluoren-9-one (M.P. 100.5-102°C) (P 359)
- 1-(N-methyl-N-methoxycarbonyl)amino-4-fluoro-fluoren-9-one (M.P. 126-128°C) (P 248)
- 1-amino-4-fluoro-fluoren-9-one (M.P. 193.5-194.5°C) (P 318)
- 1-trifluoroacetylamino-2-fluoren-9-one (M.P. 187-188°C) (P 331).

Example 35

1-(N-methyl-N-ethyl)amino-2-fluoro-fluoren-9-one(P 363)

0.5 g of 1-ethylamino-2-fluoro-fluoren-9-one are treated with 10 ml of dimethyl sulfate at 100°C during 1 hour. The excess of dimethyl sulfate is removed under vacuum.

The residue is treated with 10 ml of a 10% NaOH aqueous solution and the obtained aqueous phase is extracted with methylene chloride. The organic phase is washed with water, dried and evaporated to dryness. The obtained oil is chromatographied on silica (eluent : chloroform). 200 mg of the desired product are obtained as an oil. Yield: 37%.

Examples 36 to 37

Using the method of example 35, the following compounds have been prepared :

- 1-(N-methyl-N-isopropyl)amino-2-fluoro-fluoren-9-one (oil) (P 364)
- 1-(N-methyl-N-chloroethyl)amino-2-fluoro-fluoren-9-one (oil) (P 366).

The following examples 38 to 40 illustrate a few herbicidal compositions according to this invention.

Example 38

Wettable powder

| | |
|---|---|
| Active ingredient of formula I | 50% by weight |
| Carrier | 50% by weight |

The carrier has the following composition :

| | |
|---|---|
| - kaolin speswhite | 42% by weight |
| - colloidal silicic acid | 51% by weight |
| - surfactant | 6% by weight |
| - wetting agent | 1% by weight. |

The kaolin speswhite is sold by the "British Continental China-Clay" (Belgium), the colloidal silicic acid by "Degussa" (West Germany).

The surfactant is a sodium lignosulfonate and the wetting agent a condensate of polyoxyethylene and a fatty alcohol and are manufactured by "Tensia" (Belgium).

Example 39

Wettable powder

| | |
|---|---|
| Active ingredient of formula I | 50% by weight |
| Carrier | 50% by weight |

The carrier has the following composition :

| | |
|---|---|
| - kaolin speswhite | 92% by weight |
| - surfactant | 6% by weight |
| - wetting agent | 1% by weight |
| - colloidal silicic acid | 1% by weight. |

The characteristics of the carrier are the same as in example 38.

Example 40

Emulsifiable concentrate

Concentration : 500 g active ingredient of formula I/liter. 500 g of the active ingredient are dissolved in a liquid carrier and the solution is brought to 1 liter, the liquid carrier having the following composition for 100 ml :

- 1 g Tensiofix a.s.
- 4 g Tensiofix b.s.
- xylol to 100 ml.

The Tensiofix a.s. is an anionic surfactant, namely calcium dodecylbenzenesulfonate, and the Tensiofix b.s. is a non-ionic wetting agent, a condensate of polyoxyethylene and nonylphenol. Both are manufactured by "Tensia" (Belgium).

The following examples 41 to 56 illustrate the herbicidal activity of the compounds of formula I.

Examples 41 to 56

The selective preemergence herbicidal activity of the new derivatives of 1-amino-fluoren-9-one of formula I

is examplified by the following tests in which the seeds of several important monocotyledonous and dicotyledonous plants are separately seeded on soil in separate cups. After planting, the cups are sprayed with the selected formulations of examples 38 to 40 containing the test compound. The treated cups are then placed on greenhouse benches, watered and cared for in accordance with conventional greenhouse procedures. Three to six weeks after treatment, the tests are brought to the end and each cup is examined and rated according to a rating system expressing the percentage of injury of the considered species. The herbicidal activity of the active ingredients of the present invention is evident from the test results which are reported in the table I below.

The table gives the doses, expressed in kg of active ingredient per hectare, that has been needed to obtain an eradication of 50% and 90% of the mentioned plant species.

The tests have been performed with 1-dimethylamino-2-fluoro-fluoren-9-one as active ingredient.

TABLE I

| Ex. No. | Plant species | Doses (kg/ha) for eradication larger than 50% | 90% |
|---|---|---|---|
| 41 | Abutilon theophrasti | 2.0 | > 4.0 |
| 42 | Amaranthus hybridus | < 0.25 | < 0.25 |
| 43 | Amaranthus viridis | 0.25 | 0.5-1.0 |
| 44 | Arachis hypogea | ≫ 4.0 | ⋙ 4.0 |
| 45 | Beta vulgaris | ≪ 1.0 | ≪ 1.0 |
| 46 | Brassica napus | < 1.0 | 4.0 |
| 47 | Digitaria velutina | ≪ 0.25 | 0.25-0.5 |
| 48 | Eleusine indica | 0.25-0.50 | 1.0 |
| 49 | Gossypium hirsutum | > 8.0 | ≫ 16.0 |
| 50 | Lolium perenne | 1.0 | > 4.0 |
| 51 | Oryza sativa | > 4.0 | ≫ 4.0 |
| 52 | Panicum miliaceum | < 1.0 | 1.0-2.0 |
| 53 | Pisum sativum | ≫ 4.0 | ⋙ 4.0 |
| 54 | Setaria faberii | < 0.25 | 0.25-0.5 |
| 55 | Sorghum arundinaceum | 0.5-1.0 | > 1.0 |
| 56 | Zea mais | > 4.0 | ≫ 4.0 |

Example 57

In some cases, the mixture of one of the compounds according to this invention and another herbicide gives a synergistic mixture as examplified by the following table II.

TABLE II

A = 1-dimethylamino-2-fluoro-fluoren-9-one

B = alachlor(N-chloracetyl-N-methoxymethyl-2,6-diethyl-aniline).

| A g/ha | B g/ha | Species | % control |
|---|---|---|---|
| 250 | - | Panicum miliaeceum | 0 |
| - | 250 | " " | 30 |
| 250 | 250 | " " | 52 |
| 250 | - | Polygonum lapathifolium | 0 |
| - | 250 | " " | 20 |
| 250 | 250 | " " | 34 |
| 125 | - | Setaria faberii | 20 |
| - | 125 | " " | 80 |
| 125 | 125 | " " | 100 |
| 125 | - | Sorghum arundinaceum | 0. |
| - | 125 | " " | 26 |
| 125 | 125 | " " | 40 |

Example 58

The following table III gives the percentage of growth inhibition of various weeds in pre- and postemergence at a dose of 5 kg of active material per hectare.

TABLE III

| Active ingredient ex. | Avena sativa | | Brassica napus | | Lolium multiflorum | | Pisum sativum | |
|---|---|---|---|---|---|---|---|---|
| | PRE | POST | PRE | POST | PRE | POST | PRE | POST |
| P 221 | 0 | 0 | 0 | 100 | 0 | 30 | 0 | 0 |
| P 233 | 70 | 80 | 50 | 100 | 80 | 70 | 0 | 0 |
| P 323 | 0 | 0 | 0 | 74 | 0 | 0 | 0 | 24 |
| P 324 | 10 | 51 | 80 | 100 | 40 | 50 | 0 | 20 |
| P 325 | 44 | 66 | 68 | 100 | 72 | 86 | 10 | 50 |
| P 326 | 0 | 10 | 0 | 26 | 0 | 0 | 0 | 0 |
| P 337 | 40 | 20 | 56 | 98 | 64 | 50 | 24 | 20 |
| P 331 | 30 | 0 | 46 | 98 | 64 | 20 | 0 | 20 |
| P 333 | 0 | 0 | 30 | 98 | 30 | 30 | 0 | 0 |
| P 334 | 20 | 0 | 10 | 0 | 50 | 0 | 0 | 0 |
| P 339 | 0 | 54 | 0 | 100 | 0 | 36 | 0 | 0 |
| P 344 | 0 | 0 | 0 | 0 | 0 | 0 | 0 | 0 |
| P 348 | 0 | 98 | 60 | 100 | 50 | 100 | 0 | 60 |
| P 351 | 24 | 30 | 30 | 100 | 66 | 74 | 20 | 20 |
| P 352 | 10 | 0 | 64 | 98 | 62 | 20 | 10 | 0 |
| P 353 | 30 | 80 | 76 | 100 | 58 | 88 | 0 | 20 |
| P 349 | 0 | 0 | 50 | 100 | 10 | 26 | 20 | 0 |
| P 350 | 0 | 0 | 40 | 84 | 10 | 0 | 0 | 0 |
| P 180 | 20 | 80 | 100 | 100 | 100 | 100 | 0 | 0 |
| P 182 | 20 | 0 | 40 | 100 | 20 | 60 | 0 | 20 |
| P 194 | 0 | 0 | 0 | 0 | 0 | 0 | 0 | 0 |
| P 207 | 0 | 0 | 20 | 60 | 20 | 0 | 0 | 0 |
| P 223 | 0 | 0 | 0 | 40 | 0 | 0 | 0 | 0 |
| P 248 | 0 | 0 | 0 | 40 | 0 | 0 | 0 | 0 |
| P 318 | 36 | 20 | 56 | 100 | 58 | 40 | 0 | 20 |
| P 209 | 40 | 30 | 90 | 100 | 70 | 80 | 0 | 20 |
| P 234 | 40 | 0 | 40 | 100 | 50 | 0 | 0 | 0 |

CLAIMS

1.- New derivatives of 1-amino-fluoren-9-one represented by the following formula :

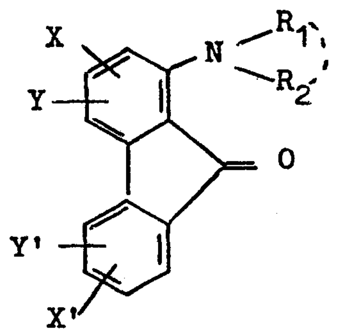

in which $R_1$ and $R_2$, which may be identical or different, represent a lower alkyl, alkanoyl, carbalcoxy, cycloalkanecarbonyl, alkenyl or alkynyl radical, which may be substituted by a halogen atom and may also form with the attached nitrogen atom a heterocyclic ring, whereas one or two of the symbols $R_1$ and $R_2$ represent hydrogen, X and X', which may be identical or different, represent hydrogen, a halogen atom, a trihalogenomethyl radical or a trifluoromethylsulfonyl ($CF_3SO_2$) radical, with the proviso that X and X' do not each represent hydrogen, and Y and Y', which may be identical or different, represent hydrogen or a nitro radical.

2. New derivatives of 1-amino-fluoren-9-one according to claim 1, in which $R_1$ and $R_2$ which may be identical or different, represent hydrogen or a lower alkyl radical and X and X', which may be identical or different, represent hydrogen or a halogen atom, with the proviso that X and X' do not each represent hydrogen, and Y as well as Y' represent hydrogen.

3. New derivatives of 1-amino-fluoren-9-one according to claims 1 and 2, in which said halogen atom is chlorine, bromine, iodine or fluorine.

4. New derivatives of 1-amino-fluoren-9-one according to claims 1 and 2, in which said trihalogenomethyl radical is selected from the group consisting of trifluoromethyl, trichloromethyl and tribromomethyl radical.

5. 1-amino-2-fluoro-fluoren-9-one.

6. 1-diloweralkylamino-2-fluoro-fluoren-9-ones.

7. 1-dimethylamino-2-fluoro-fluoren-9-one.

8. Herbicidal compositions containing a herbicidally effective amount of at least one compound according to claim 1 or 2, together with a carrier or adjuvant.

9. Herbicidal compositions containing, as active ingredient, a herbicidally effective amount of at least one compound selected from the group consisting of 1-amino-2-fluoro-fluoren-9-one, 1-diloweralkylamino-2-fluoro-fluoren-9-ones and 1-dimethylamino-2-fluoro-fluoren-9-one, together with a carrier or adjuvant.

10. Herbicidal compositions containing a herbicidally effective amount of 1-dimethylamino-2-fluoro-fluoren-9-one as active ingredient together with a carrier or adjuvant.

11. Herbicidal compositions containing at least one compound according to claim 1 or 2 in admixture with known herbicides, said admixture being in a herbicidally effective amount.

12. Method for the preemergence or postemergence control of weeds consisting in applying to soil containing weeds a herbicidally effective amount of at least one herbicidal composition according to claims 8, 9, 10 or 11.

13. Method for the preemergence or postemergence control of weeds consisting in applying to soil containing weeds a herbicidally effective amount of 1-dimethylamino-2-fluoro-fluoren-9-one.

European Patent Office

# EUROPEAN SEARCH REPORT

Application number

EP 79 200 181.0

| Category | Citation of document with indication, where appropriate, of relevant passages | Relevant to claim | CLASSIFICATION OF THE APPLICATION (Int. Cl.²) |
|---|---|---|---|
| X | BE - A - 666 353 (MANUFACTURE DE PRODUITS PHARMACEUTIQUES A. CHRISTIAENS) * claims 10, 12, 13 * | 1-3 | C 07 C 97/07<br>A 01 N 9/20<br>C 07 C 103/42<br>C 07 C 147/06<br>C 07 C 147/12<br>C 07 D 295/10 |
| X | GB - A - 1 197 054 (MANUFACTURE DE PRODUITS PHARMACEUTIQUES A. CHRISTIAENS) * formula III, examples 36 to 38, 50 * | 1-3 | |
| X | US - A - 3 296 252 (SANDOZ) * examples 2 and 3 * | 1-3 | **TECHNICAL FIELDS SEARCHED (Int.Cl.²)**<br>A 01 N 9/20<br>C 07 C 97/00<br>C 07 C 103/42<br>C 07 C 147/00<br>C 07 D 295/10 |
| X | Chemical Abstracts, Volume 67, Nr. 23, December 4, 1967, (COLUMBUS, OHIO, USA) K.A. MAIER et al. "Halogenation of 1-aminofluoren-9-one" page 10992, column 2, abstract Nr. 116738j & Monatshefte Chem., Volume 98, Nr. 4, 1967 pages 1567 to 1576 | 1-3 | |

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| Berlin | 24-07-1979 | KAPTEYN |